# EUROPEAN PATENT APPLICATION

(11) **EP 2 905 733 A1**
(43) Date of publication of application: **12.08.2015**
(21) Application number: 15153547.3
(22) Date of filing: 03.02.2015
(51) Int. Cl.: G06Q 10/10, G06Q 50/18

(54) **System and method for digital or electronic power of attorney service**

(30) Priority: 10.02.2014 US 201414176947
(71) Applicant: Ims Health Incorporated, Danbury, CT 06810 (US)
(72) Inventor: Mahgoub, Hussam, Toronto, Ontario M6P 2S5 (CA); Blair, Charles, Toronto, Ontario M2N 6M4 (CA); Florez, Elkin, Richmond Hills, Ontario L4C 08T (CA); Fung, Ryan, New York, Ontario M2N 6Z6 (CA)
(74) Representative: Casey, Alan Michael

(57) **Abstract**

System and method to process a digital power of attorney (DPOA), the method including: receiving a request from the grantor to create a DPOA to name a predetermined grantee; transmitting the request to an authentication server in order to authenticate the request; receive an authentication result from the authentication server; and if the authentication server is positive, issuing the DPOA to the predetermined grantee. The method may further include receiving a request from a purported grantee or from a digital service provider to exercise the DPOA; authenticating, by the authentication server, an identity of the purported grantee; if the purported grantee is the predetermined grantee, verifying a condition of usage of the DPOA; and if the condition is verified, granting a power specified by the DPOA.

## Description

### BACKGROUND

### Field of the Invention

Embodiments of the present invention generally relate to the authentication of powers of attorney, and, in particular, to a system and method for automated validation, usage and management of a digital or electronic power of attorney authorization in a digital service environment.

### Description of Related Art

Access and/or control related to certain sensitive information such as financial information (e.g., banking accounts, tax information, and the like), health information (e.g., hospital records, doctor records, health-care decisions, etc.) and the like, ordinarily may be restricted only to the person to whom the information or control pertains to, for reasons of privacy or as a matter of law. Access or control by other persons may require a court order, e.g., arising from probate of an estate, a competency hearing, a lawsuit, a criminal investigation, and so forth.

A legal instrument known as a power of attorney (POA) conventionally is a written authorization for a first, designated person to represent or act on behalf of a second person in private affairs, business, or some other legal matter, sometimes against the wishes of the second person. The person authorizing the other to act is termed the principal, grantor, or donor (of the power). The person receiving this power and authorized to act is termed the grantee, agent, or attorney-in-fact. The POA allows a grantor to designate in advance a grantee who is authorized under conditions described in the POA to act on behalf of and in the legal capacity for the grantor. Such authorization may be a result of death, incapacity, or other designated triggering conditions.

The POA is ordinarily a physical paper document with indicia of authenticity, such as an embossed seal, a notary's seal and/or signature, and so forth. Even with such indicia, the POA is often inspected for signs of tampering (e.g., inserted or removed pages). However, such physical documents are generally limited in flexibility, speed of usage and ease of use. Human attorneys and court authorization may be needed in order to make them effective, leading to inefficiencies and increased costs.

Although a conventional POA as described above is suitable for conventional financial accounts and health records, such information is increasingly maintained only in digital form, accessed primarily or exclusively by electronic or online methods. Furthermore, additional forms of information are increasingly used, such as digital assets in cloud storage, digital commerce records, social media relationships, digital files (e.g., in DropBox), alternative digital currencies and/or wallets (e.g., BITCOIN, Amazon Coin), electronic health records, and the like. Conventional POAs are less suitable to these more recent forms of information, information storage, and information access. For example, in situations contemplated by a PAO, accessing digital files stored in the cloud may involve a relatively lengthy process to contact a storage provider, give them a hard copy of the POA, and wait to be granted access and/or resort to court relief if access is not granted at first. This is a very lengthy process compared to the normal access time to the information in the absence of the need for a POA.

Another related problem is in the area of digital code signing of digital objects or content such as software, documents, digitized audio/video/image data. For example, digital code signing may be used for digital verification that software is genuine and has not been tampered with between the time it was produced by a developer and the time a consumer receives it for installation and/or usage. Additional uses of digital signing would involve assuring documents are genuine, that music or video files are properly licensed, and so forth. Currently, the developer of software needs to perform the digital code signing, which is complicated process. Known application development platforms do not enable the transfer of rights to sign and represent a grantor to third parties.

Other known systems may include public-key infrastructure (PKI)/ digital certificates, attribute/ authorization certificates and digital rights management (DRM). However, PKI/ digital certificates provide only strong authentication and digital signature services. Attribute/authorization certificates provide access control service only to third parties. DRM provides control for digital media after sales. There is no comprehensive and automated solution.

Therefore, a need exists to provide a digital process and/or system to facilitate the authorization and transferring of access rights and ownership of digital assets to third parties. Furthermore, there exists a need for a more streamlined process for a developer of digital code, object, content or the like (developed using a predetermined development platform) to authorize the development platform to digitally sign the digital code, object, content or the like, on behalf of the developer.

### SUMMARY

In one embodiment, a method to process a digital power of attorney (DPOA) may include: receiving a request from the grantor to create a DPOA to name a predetermined grantee; transmitting the request to an authentication server in order to authenticate the request; receive an authentication result from the authentication server; and if the authentication server is positive, issuing the DPOA to the predetermined grantee. The method may further include receiving a request from a purported grantee to exercise the DPOA; authenticating, by the authentication server, an identity of the purported grantee; if the purported grantee is the predetermined grantee, verifying a condition of usage of the DPOA; and if the condition is verified, granting a power specified by the DPOA.

In one embodiment, a system to process a digital power of attorney (DPOA) may include: a DPOA issuer comprising a processor coupled to a memory, the DPOA issuer configured to issue a DPOA upon request of a grantor; a receiver configured to receive a request from the grantor to create a DPOA to name a predetermined grantee; a communication interface to an authentication server, the authentication server configured to authenticate the request; and a transmitter configured to issue the DPOA to the predetermined grantee in response to an authenticated request. The system may further include a receiver configured to receive a request from the predetermined grantee to exercise the DPOA; an authentication module configured to authenticate an identity of the predetermined grantee; and a verification module configured to verify a condition of usage of the DPOA and grant a power specified by the DPOA.

The preceding is a simplified summary of embodiments of the disclosure to provide an understanding of some aspects of the disclosure. This summary is neither an extensive nor exhaustive overview of the disclosure and its various embodiments. It is intended neither to identify key or critical elements of the disclosure nor to delineate the scope of the disclosure but to present selected concepts of the disclosure in a simplified form as an introduction to the more detailed description presented below. As will be appreciated, other embodiments of the disclosure are possible utilizing, alone or in combination, one or more of the features set forth above or described in detail below.

### BRIEF DESCRIPTION OF THE DRAWINGS

The above and still further features and advantages of the present invention will become apparent upon consideration of the following detailed description of embodiments thereof, especially when taken in conjunction with the accompanying drawings wherein like reference numerals in the various figures are utilized to designate like components, and wherein:
FIG. 1 is a block diagram depicting a mobile communications network in accordance with an embodiment of the present invention;
FIG. 2A is a system level block diagram depicting an end-user mobile device in accordance with an embodiment of the present invention;
FIG. 2B is a system level block diagram depicting an end-user non-mobile device in accordance with an embodiment of the present invention; and
FIG. 3 illustrates, at a high level of abstraction, a process in accordance with an embodiment of the present invention.

The headings used herein are for organizational purposes only and are not meant to be used to limit the scope of the description or the claims. As used throughout this application, the word "may" is used in a permissive sense (*i.e.,* meaning having the potential to), rather than the mandatory sense (*i.e.,* meaning must). Similarly, the words "include", "including", and "includes" mean including but not limited to. To facilitate understanding, like reference numerals have been used, where possible, to designate like elements common to the figures. Optional portions of the figures may be illustrated using dashed or dotted lines, unless the context of usage indicates otherwise.

### DETAILED DESCRIPTION

The disclosure will be illustrated below in conjunction with an exemplary digital information system. Although well suited for use with, e.g., a system using a server(s) and/or database(s), the disclosure is not limited to use with any particular type of system or configuration of system elements. Those skilled in the art will recognize the disclosed techniques may be used in any system or process in which it is desirable to provide a transferable permission to access information or control a decision.

The exemplary systems and methods of this disclosure will also be described in relation to software, modules, and associated hardware. However, to avoid unnecessarily obscuring the present disclosure, the following description omits well-known structures, components and devices that may be shown in block diagram form, are well known, or are otherwise summarized.

In the following detailed description, numerous specific details are set forth in order to provide a thorough understanding of embodiments or other examples described herein. In some instances, well-known methods, procedures, components and circuits have not been described in detail, so as to not obscure the following description. Further, the examples disclosed are for exemplary purposes only and other examples may be employed in lieu of, or in combination with, the examples disclosed. It should also be noted the examples presented herein should not be construed as limiting of the scope of embodiments of the present invention, as other equally effective examples are possible and likely.

As used herein, the term "module" refers generally to a logical sequence or association of steps, processes or components. For example, a software module may comprise a set of associated routines or subroutines within a computer program. Alternatively, a module may comprise a substantially self-contained hardware device. A module may also comprise a logical set of processes irrespective of any software or hardware implementation.

As used herein, the term "transmitter" may generally comprise any device, circuit, or apparatus capable of transmitting a signal. As used herein, the term "receiver" may generally comprise any device, circuit, or apparatus capable of receiving a signal. As used herein, the term "transceiver" may generally comprise any device, circuit, or apparatus capable of transmitting and receiving a signal. As used herein, the term "signal" may include one or more of an electrical signal, a radio signal, an optical signal, an acoustic signal, and so forth.

The term "computer-readable medium" as used herein refers to any tangible storage and/or transmission medium that participates in storing and/or providing instructions to a processor for execution. Such a medium may take many forms, including but not limited to, non-volatile media, volatile media, and transmission media. Non-volatile media includes, for example, NVRAM, or magnetic or optical disks. Volatile media includes dynamic memory, such as main memory. Common forms of computer-readable media include, for example, a floppy disk, a flexible disk, hard disk, magnetic tape, or any other magnetic medium, magneto-optical medium, a CD-ROM, any other optical medium, punch cards, paper tape, any other physical medium with patterns of holes, RAM, PROM, EPROM, FLASH-EPROM, solid state medium like a memory card, any other memory chip or cartridge, a carrier wave as described hereinafter, or any other medium from which a computer can read. A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/or the like. Accordingly, the disclosure is considered to include a tangible storage medium or distribution medium and prior art-recognized equivalents and successor media, in which the software implementations of the present disclosure are stored.

Embodiments in accordance with the present disclosure may provide a digital process and/or system to facilitate the authorization and transferring of access rights and ownership or control over of digital assets to third parties. Furthermore, embodiments may provide a more streamlined process for a developer of digital code, object, content or the like, developed using a predetermined development platform, to authorize the development platform to digitally sign the digital code, object, content or the like, on behalf of the developer. Unless the content is digitally signed, the content may be untrusted. Digitally signing the content may also be useful in enforcing access restrictions until a user can be authorized.

More particularly, embodiments in accordance with the present disclosure may provide an electronic service to facilitate use of a power of attorney (POA) legal instrument when needed in order to access digital assets, transfer the digital rights of digital and facilitate electronic transactions. Such electronic service may be referred to as a digital POA (DPOA). DPOA services may also include an authorization for a third party designated in the DPOA to digitally sign on behalf of the requester. DPOA powers may be granted temporarily or permanently.

Embodiments in accordance with the present disclosure provide a system and method to provide and use a DPOA. Embodiments may include a trusted issuer system that issues an electronic DPOA upon a request from a grantor. Embodiments may further include a trusted validation system that validates electronic DPOA requests from purported grantees. A grantor and/or a grantee may be a human user or another electronic system or service (e.g., an electronic system implementing an artificial intelligence process).

An electronic DPOA may consist of digital signature of the issuer and/or other information such as requester information (e.g., information about the grantor including strong authentication information), third party information (information about the grantee including strong authentication information), scope of the DPOA or other service, and time period during which the DPOA is effective.

A DPOA may be issued only after usage of a strong authentication process in order to authenticate a grantor to above a predetermined level of confidence, in order to verify a scope of service over which the DPOA may be effective, in order to verify third party information (e.g., a proof of identify of a digital data store) and in order to provide proof of ownership of the data stored in the data store. A DPOA may be issued if the issuance request is found to be valid.

A request from a grantee to use the powers or access the data granted by the DPOA may include a strong authentication technique in order to authenticate a grantee to above a predetermined level of confidence, verification of a scope over which the DPOA may be valid, verification of a scope over which the DPOA may be effective, and verification that the present conditions at least partially satisfy conditions for the DPOA to become effective. If the DPOA is determined to be both valid and effective, then the grantee and/or the digital data store may be notified that the DPOA may be used by the grantee. Otherwise, grantee and/or the digital data store may be notified that the DPOA may not be used by the grantee.

Computing devices including mobile computing devices have become ubiquitous. Many users have even cancelled traditional landline telephone services at their residences and/or businesses, and have adopted mobile phones as their primary means of communications. Accordingly, many users typically carry such mobile devices with them wherever they go. For purposes of the discussion herein, mobile devices may include smart phones, mobile telephones, personal digital assistants, and other portable computing devices that have a network communications interface and an output interface, such as a display. Mobile devices may include a subscriber identification module ("SIM") card that can provide additional capabilities and/or capacity. By enabling users to access, manipulate, communicate and/or store digital data of various forms, some of which may need to be authenticated, such mobile devices allow users to access sensitive or otherwise protected data, and/or to access digital content that initially may be untrusted or unauthorized until the digital content can be verified or the user can be authorized.

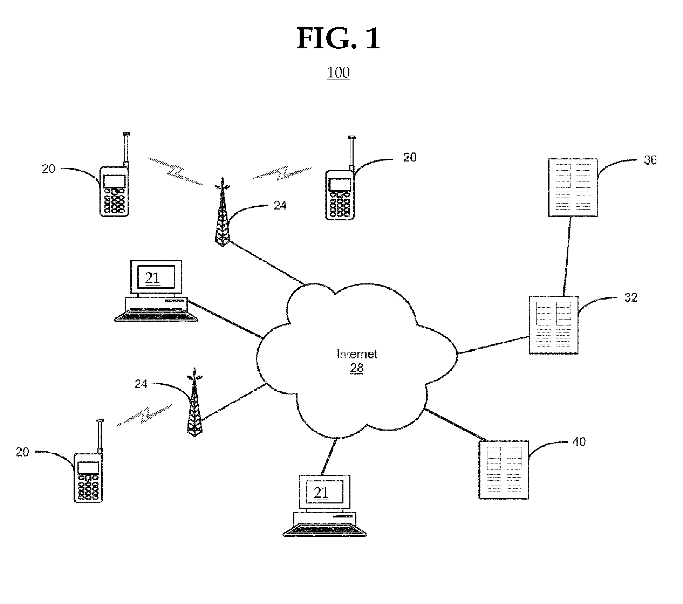

FIG. 1 illustrates a system for transferring an authorization via computing devices and its operating environment in accordance with an embodiment of the invention. The embodiment described herein relates to a system for transferring an authorization such as a DPOA between two parties: in this case, a grantor and a grantee. At least the grantee may be equipped with a computing device, more specifically a computing device that may have an authorization transfer application installed on it. The authorization transfer application may be a custom application or a standard web browser.

A number of mobile devices 20 are shown in communication wirelessly with cellular base stations 24 via cellular communications. The cellular base stations 24 enable communications over a large, public network, such as the Internet 28, via a number of intermediate servers operated by one or more cellular communications carriers (not shown). FIG. 1 further illustrates a number of non-mobile computing devices 21 in communicative contact with Internet 28. Mobile devices 20 and non-mobile devices 21 herein may be referred to individually or collectively as computing devices. A digital POA issuer and validation server 32 (also referred to herein as "DPOA issuer 32") may also be in communication with the Internet 28. The DPOA issuer 32 may also be in communication with an authentication server 36 over a network. In some embodiments, authentication server may be an external service or third-party. Additionally, the DPOA issuer 32 may be in communication with one or more repositories of sensitive information such as a financial institution 40 where the users of the mobile devices 20 and/or non-mobile devices 21 may have a relationship of some sort, such as business or health care. In some embodiments DPOA issuer 32, authentication server 36 and/or financial institution 40 may be implemented as a single computing device. In other embodiments DPOA issuer 32, authentication server 36 and/or financial institution 40 may be implemented by two or more computing devices that are communicatively coupled to each other.

Embodiments in accordance with the present disclosure are not limited to the types of mobile devices 20 and/or non-mobile devices 21 illustrated in FIG. 1. Embodiments may be used with substantially any type of input/output device or terminal including PCs, MacBooks, tablet computer, thin clients, or substantially any other type of computing device accessible via a network.

It should be emphasized the configuration of the elements as shown in FIG. 1 is for purposes of illustration only and should not be construed as limiting embodiments of the present invention to any particular arrangement of elements.

The server may be a software-controlled system including a processing unit (CPU), microprocessor, or other type of digital data processor executing software or an Application-Specific Integrated Circuit (ASIC) as well as various portions or combinations of such elements. The memory may comprise random access memory (RAM), a read-only memory (ROM), or combinations of these and other types of electronic memory devices. Embodiments of the present invention may be implemented as software, hardware (such as, but not limited to, a logic circuit), or a combination thereof.

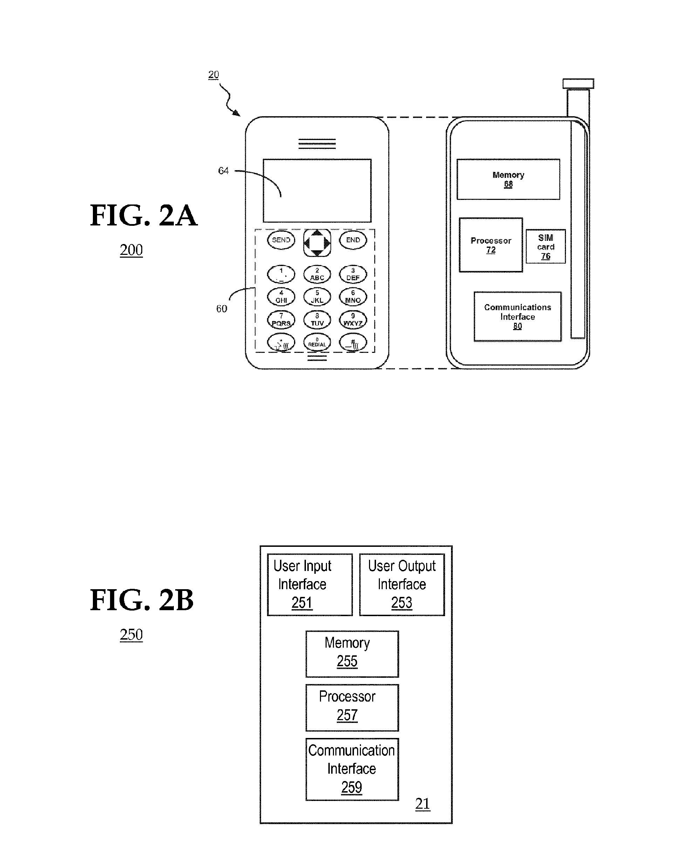

Referring to FIG. 2A, a number of components of mobile device 20 are shown. As illustrated, in this embodiment, mobile device 20 is a typical mobile phone having basic functions. Mobile device 20 has an input interface 60 for receiving input from a user, and a display 64 is provided for presenting information visually to the user. Mobile device 20 also includes memory 68 for storing an operating system that controls the main functionality of mobile device 20, along with a number of applications that are run on mobile device 20, and data. A processor 72 executes the operating system and applications. A SIM card 76 provides additional memory for storing applications and data, and has a microprocessor for executing them. Additionally, SIM card 76 has a unique hardware identification code that permits identification of mobile device 20. When installed, SIM card 76 forms part of mobile device 20. Other types of mobile devices can have encrypted device memory in place of SIM card 76 that offers the equivalent functionality. A communications interface 80 permits communications with a cellular network for voice and data.

Referring to FIG. 2B, a number of components of non-mobile computing device 21 are shown. As illustrated, in this embodiment, non-mobile device 21 is a typical desktop or tower computer having basic functions. Non-mobile device 21 has a user input interface 251 for receiving input from a user (e.g., a keyboard, touchscreen and/or microphone), and a user output interface 253 is provided for presenting information visually or audibly to the user. Non-mobile device 21 also includes memory 255 for storing an operating system that controls the main functionality of non-mobile device 21, along with a number of applications that are run on non-mobile device 21, and data. A processor 257 executes the operating system and applications. Non-mobile device 21 may have a unique hardware identification code that permits identification of non-mobile device 21 (e.g., a medium access control (MAC) address). At least a portion of memory 255 may be encrypted. A communications interface 259 permits communications with a LAN or Internet 28, e.g., by way of an Ethernet or Wi-Fi interface.

In order to enable mobile device 20 or non-mobile computing device 21 to transfer or receive authorizations in the system, a grantor using mobile device 20 or non-mobile computing device 21 may register with the DPOA issuer, e.g., via a webpage on mobile device 20 or non-mobile computing device 21 or elsewhere. During registration, the user may provide identification information concerning himself, the grantee, under what circumstances the grantee may receive authorization, and/or sufficient information to access the accounts pertaining to the grantor, e.g., account number, password or other indicia of access permission to the data, and so forth. Once registration is complete, the account may be enabled for a future transfer of a DPOA to a grantee who may also be using a similar mobile device 20 or non-mobile computing device 21. The DPOA when invoked may be accessed (e.g., downloaded) either directly by mobile device 20 or non-mobile computing device 21.

Once the DPOA is installed on, the authorization transfer application may direct the user to enter strong identification information (e.g., username, password, PIN, digital certificates, a one-time password, biometrics such as fingerprint or iris scan, and so forth).

After the setup procedure, the transfer authorization application is able to present options to, and receive input from, the user, and carry out communications over Internet 28 via communications interface 80 or 259 of mobile device 20 or non-mobile computing device 21, respectively.

Once the transfer authorization application has been installed and configured on mobile device 20 or non-mobile computing device 21, the device can be used to authorize transfers.

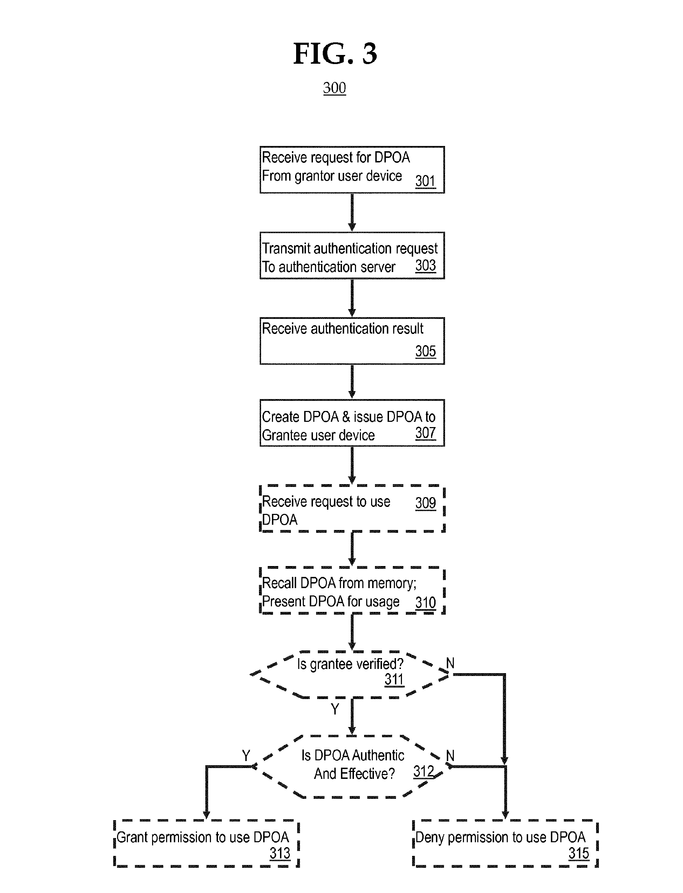

FIG. 3 illustrates at a high level of abstraction a process 300 in accordance with an embodiment of the present disclosure. Process 300 may be useful when a grantor arranges to allow a grantee to have future access and/or control over a digital asset owned or controlled by the grantee. Process 300 begins at step 301, at which DPOA issuer 32 may receive a request from the grantor operating from a communication terminal such as mobile device 20 or non-mobile device 21 to issue a DPOA. The grantor may use other kinds of communication terminals such as a fixed (i.e., desktop) communication terminal, a conventional telephone with touch-tone interface, and so forth. As part of the request, the grantor may present sufficient credentials to verify their identity. ID verification is a sub process that may include a purported grantor presenting sufficient credentials (e.g., user names, passwords, answers to challenge questions, machine-ID information, cookie information, biometric information, etc.) to DPOA issuer 32 and/or authentication server 36 in order to determine above a predetermined threshold of certainty that the purported grantor is in-fact the actual grantor.

The request may limit the scope of the DPOA to one or more of: a predetermined grantee; a predetermined digital asset or set of digital assets that the DPOA applies to (e.g., a bank account); a predetermined type of control decisions that the DPOA applies to (e.g., health care decisions); a predetermined authorized action or set of authorized actions under the DPOA (e.g., withdrawing funds from a bank account, termination of life support, etc.); a predetermined time window of validity; a predetermined date/time at which the DPOA becomes valid; a predetermined date/time at which the DPOA expires; a predetermined triggering condition or set of triggering conditions upon which the DPOA powers may be exercised (e.g., upon being found incompetent by a court, upon a grantor being missing more than a predetermined amount of time; upon a grantor being declared legally dead, upon being comatose in a hospital, etc.); and so forth.

Next, control of process 300 transitions to step 303, at which DPOA issuer 32 may transmit an authentication request to authentication server 36. The authentication request may include information such as credentials of the grantor, identity of the grantee, and scope and conditions under which the DPOA is to become effective.

Next, control of process 300 transitions to step 305, at which DPOA issuer 32 may receive an authentication result from authentication server 36 in response to the authentication request. If the authentication result is negative, process 300 may end.

Next, control of process 300 transitions to step 307, at which if the authentication result from step 305 is positive then DPOA issuer 32 may create a DPOA and transmit the DPOA to a user device. The user device may be a user device of the grantee, or alternatively the user device may be a user device of the grantor, who in turn will later distribute the DPOA to the grantee. In some embodiments, DPOA issuer 32 may store the DPOA in a secure memory directly accessible by DPOA issuer 32. In some embodiments, the grantor and/or grantee may be notified of the creation of the DPOA but may not receive the DPOA itself.

In some embodiments, the DPOA may include the scope, conditions or other limitations set by the grantor. Doing so may improve portability of the DPOA. The grantee may store, in a memory accessible to the grantee, a copy of the DPOA until it is needed. For example, the DPOA may be stored in a memory of mobile device 20 or non-mobile computing device 21.

Next, process 300 turns to optional steps at a later point in time, possibly a distant point in the future, to execute if and when a purported grantee may decide or need to use the DPOA. Control of process 300 may transition to step 309 at which the purported grantee may request to use the DPOA. Next, control of process 300 may transition to step 310 at which the purported grantee will recall the DPOA from a memory and present the DPOA to a digital service (e.g., an electronic interface of a bank or custodian such as financial institution 40, or an electronic interface of a health care provider, etc.). The presentation will ordinarily be via a communication network such as internet 28. The request includes identification credentials of the purported grantee.

Next, control of process 300 may transition to step 311 at which the identity of the purported grantee may be verified. The digital service (e.g., financial institution 40) routes the request to the DPOA issuer 32 which validates the grantee credentials with the authentication server 36. Authentication of the purported grantee may take place in a similar manner as authentication of the purported grantor by the authentication server 36. If the identity of the purported grantee is not verified, then control of process may transition to step 315 at which permission to use the DPOA is denied.

After successful authentication in step 311, the identity of the purported grantee is authenticated as being the identity of the grantee, but the grantee's authority to act in this specific situation has not yet been confirmed. Confirming an authority to act may include confirming that a condition of usage of the DPOA is satisfied.

Next, control of process 300 transitions to step 312, at which the DPOA issuer/validation is evaluating the authenticity and effectiveness of the DPOA in order to verify that the DPOA is genuine, that the DPOA is currently effective, and/or that the grantee is requesting control and/or access that is within the scope of the DPOA. If the evaluation is positive, then control of process 300 passes to step 313 at which the grantee has permission to use the DPOA, e.g., the grantee is allowed access to the grantor's digital asset, or allowed to exercise control within the authority of the DPOA. The digital service provider (e.g., financial institution 40) may set limits or otherwise monitor the grantee's activities in order to ensure that the grantee remains within the limits of authority of the DPOA.

If the response from DPOA issuer/validation 32 is negative then process 300 passes to step 315 at which the grantee is denied permission to use the DPOA. The grantee may be informed that the request to act or to access under color of the DPOA has been denied.

In some circumstances, it may not be possible to automatically verify all predicate conditions for the DPOA to be currently effective. For example, if the grantee is acting under color of the DPOA due to a medical condition such as the grantor being comatose for more than a predetermined amount of time, the DPOA issuer/validation server 32 may provide a conditional positive DPOA approval, subject to an additional approval or validation such as by a human such as an attending physician. If such conditions spontaneously may no longer be true (e.g., a patient may come out of a coma, or a missing person may be found), the conditional positive DPOA approval may be set to expire after a predetermined time (e.g., 24 hours), but subject to renewal.

Embodiments of the present invention include a system having one or more processing units coupled to one or more memories. The one or more memories may be configured to store software that, when executed by the one or more processing unit, allows practice of embodiments described herein, including at least in FIG. 3 and related text.

The disclosed methods may be readily implemented in software, such as by using object or object-oriented software development environments that provide portable source code that can be used on a variety of computer or workstation platforms. Alternatively, the disclosed system may be implemented partially or fully in hardware, such as by using standard logic circuits or VLSI design. Whether software or hardware may be used to implement the systems in accordance with various embodiments of the present invention may be dependent on various considerations, such as the speed or efficiency requirements of the system, the particular function, and the particular software or hardware systems being utilized.

While the foregoing is directed to embodiments of the present invention, other and further embodiments of the present invention may be devised without departing from the basic scope thereof. It is understood that various embodiments described herein may be utilized in combination with any other embodiment described, without departing from the scope contained herein. Further, the foregoing description is not intended to be exhaustive or to limit the invention to the precise form disclosed. Modifications and variations are possible in light of the above teachings or may be acquired from practice of the invention. Certain exemplary embodiments may be identified by use of an open-ended list that includes wording to indicate that the list items are representative of the embodiments and that the list is not intended to represent a closed list exclusive of further embodiments. Such wording may include "e.g.," "etc.," "such as," "for example," "and so forth," "and the like," etc., and other wording as will be apparent from the surrounding context.

No element, act, or instruction used in the description of the present application should be construed as critical or essential to the invention unless explicitly described as such. Also, as used herein, the article "a" is intended to include one or more items. Where only one item is intended, the term "one" or similar language is used. Further, the terms "any of" followed by a listing of a plurality of items and/or a plurality of categories of items, as used herein, are intended to include "any of," "any combination of," "any multiple of," and/or "any combination of multiples of" the items and/or the categories of items, individually or in conjunction with other items and/or other categories of items.

Moreover, the claims should not be read as limited to the described order or elements unless stated to that effect. In addition, use of the term "means" in any claim is intended to invoke 35 U.S.C. §112, 6, and any claim without the word "means" is not so intended.

## Claims

1. A method to process a digital power of attorney (DPOA), comprising:
receiving a request from the grantor to create a DPOA to name a predetermined grantee, the request comprising grantor authentication credentials;
transmitting the grantor authentication credentials to an authentication server in order to authenticate the grantor;
receive an authentication result from the authentication server; and
if the authentication server is positive, issuing the DPOA to the predetermined grantee.

2. The method of claim 1, further comprising:
receiving a request from a purported grantee to exercise the DPOA;
authenticating, by the authentication server, an identity of the purported grantee;
if the purported grantee is the predetermined grantee, verifying a condition of usage of the DPOA; and
if the condition is verified, granting a power specified by the DPOA.

3. The method of claim 1, wherein authenticating comprises usage of biometric information, digital certificates, passwords, one time password or other predetermined authentication credentials.

4. The method of claim 1, wherein scope of the DPOA comprises one or more of identification of a predetermined digital asset that the DPOA applies to, identification of a predetermined type of control decisions that the DPOA applies to, identification of a predetermined authorized action, a predetermined time period and a predetermined triggering condition.

5. The method of claim 1, wherein scope of the DPOA is stored with the DPOA.

6. The method of claim 1, wherein scope of the DPOA is stored on the DPOA issuer/validation server.

7. The method of claim 1, wherein power specified by the DPOA comprises one or more of power over a financial account, permission to use a digital object and power over health care treatment of another person.

8. The method of claim 1, further comprising: providing, by the DPOA issuer/validation server, a conditional approval subject to an additional approval of DPOA usage by a human approver.

9. The method of claim 1, further comprising: monitoring, by the DPOA issuer/validation server, compliance by the grantee to scope of the DPOA.

10. A system to process a digital power of attorney (DPOA), comprising:
a DPOA issuer comprising a processor coupled to a memory, the DPOA issuer configured to issue a DPOA upon request of a grantor;
a receiver configured to receive a request from the grantor to create a DPOA to name a predetermined grantee;
a communication interface to an authentication server, the authentication server configured to authenticate the grantor or grantee identity; and
a transmitter configured to issue the DPOA to the predetermined grantee in response to an authenticated request.

11. The system of claim 10, further comprising:
a receiver configured to receive a request from the predetermined grantee to exercise the DPOA;
an authentication module configured to authenticate an identity of the predetermined grantee; and
a verification module configured to verify a condition of usage of the DPOA and grant a power specified by the DPOA.

12. The system of claim 11, wherein the verification module is further configured to provide a conditional approval subject to an additional approval of DPOA usage by a human approver.

13. The system of claim 10, wherein scope of the DPOA comprises one or more of identification of a predetermined digital asset that the DPOA applies to, identification of a predetermined type of control decisions that the DPOA applies to, identification of a predetermined authorized action, a predetermined time period and a predetermined triggering condition.

14. The system of claim 10, further comprising an interface to a monitoring module, the monitoring module configured to monitor compliance by the grantee to scope of the DPOA.

15. The system of claim 10, wherein the power specified by the DPOA comprises one or more of power over a financial account, permission to use a digital object and power over health care treatment of another person.
